# EUROPÄISCHE PATENTANMELDUNG

(11) **EP 0 680 951 A2**
(43) Veröffentlichungstag der Anmeldung: **08.11.1995**
(21) Anmeldenummer: 95105853.6
(22) Anmeldetag: 19.04.1995
(51) Int. Cl.: C07D 209/46, C07D 217/24

(54) **Schwefelhaltige Verbindungen**

(30) Priorität: 28.04.1994 DE 44148801
(71) Anmelder: BASF AKTIENGESELLSCHAFT, D-67056 Ludwigshafen (DE)
(72) Erfinder: Patsch, Manfred, Dr., D-67157 Wachenheim (DE); Schefczik, Ernst, Dr., D-67069 Ludwigshafen (DE)

(57) **Zusammenfassung**

Schwefelverbindungen der Formel 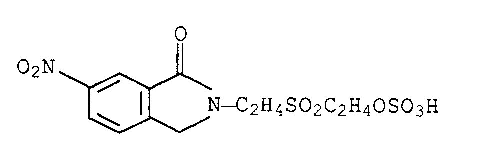
in der
- n: 0 oder 2,
- R¹ und R²: Wasserstoff, C₁-C₆-Alkyl, C₁-C₆-Alkoxy, Halogen, Nitro, Amino, Hydroxysulfonyl, C₁-C₆-Alkoxycarbonyl oder gegebenenfalls substituiertes Carbamoyl,
- A: Methylen, Carbonyl, Sulfonyl oder einen Rest der Formel CH₂―CO oder CH₂-SO₂,
- X: eine direkte Bindung oder gegebenenfalls substituiertes C₁-C₈-Alkylen und
- Y: Vinyl oder einen Rest der Formel C₂H₄Q, worin Q für Hydroxy oder eine unter alkalischen Reaktionsbedingungen abspaltbare Gruppe steht,
bedeuten, sowie ein Verfahren zu deren Herstellung.

## Beschreibung

Die vorliegende Erfindung betriffft neue Schwefelverbindungen der Formel I 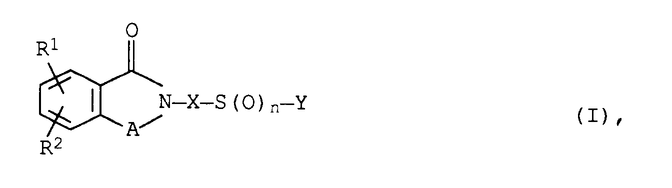
in der
- n: 0 oder 2,
- R¹ und R²: unabhängig voneinander jeweils Wasserstoff, C₁-C₆-Alkyl, C₁-C₆-Alkoxy, Halogen, Nitro, Amino, Hydroxysulfonyl, C₁-C₆-Alkoxycarbonyl, Carbamoyl oder C₁-C₆-Mono- oder Dialkylcarbamoyl,
- A: Methylen, Carbonyl, Sulfonyl oder einen Rest der Formel CH₂―CO oder CH₂-SO₂, wobei die Methylengruppe jeweils an den Benzolring geknüpft ist,
- X: eine direkte Bindung, C₁-C₈-Alkylen, das gegebenenfalls durch 1 bis 3 Sauerstoffatome in Etherfunktion, 1 bis 3 Iminogruppen oder 1 bis 3 C₁-C₄-Alkyliminogruppen unterbrochen ist, oder einen Rest der Formel L¹―CO―NR³―L², worin L¹ und L² unabhängig voneinander jeweils für C₁-C₄-Alkylen und R³ für Wasserstoff, C₁-C₄-Alkyl oder Phenyl stehen, und
- Y: Vinyl oder einen Rest der Formel C₂H₄Q, worin Q für Hydroxy oder eine unter alkalischen Reaktionsbedingungen abspaltbare Gruppe steht,
bedeuten, sowie ein Verfahren zu ihrer Herstellung.

Aufgabe der vorliegenden Erfindung war es, neue Schwefelverbindungen bereitzustellen, die einen Benzamid-Strukturteil aufweisen. Die neuen Stoffe sollten sich in vorteilhafter Weise zur Herstellung von Farbstoffen eignen.

Demgemäß wurden die eingangs näher bezeichneten Schwefelverbindungen der Formel I gefunden.

Alle in der obengenannten Formel auftretenden Alkyl- und Alkylengruppen können sowohl geradkettig als auch verzweigt sein.

Reste R¹, R² und R³ sind z.B. Methyl, Ethyl, Propyl, Isopropyl, Butyl, Isobutyl, sec-Butyl oder tert-Butyl.

Reste R¹ und R² sind weiterhin z.B. Pentyl, Isopentyl, Neopentyl, tert-Pentyl, Hexyl, 2-Methylpentyl, Methoxy, Ethoxy, Propoxy, Isopropoxy, Butoxy, Isobutoxy, sec-Butoxy, tert-Butoxy, Pentyloxy, Isopentyloxy, Neopentyloxy, tert-Pentyloxy, Hexyloxy, 2-Methylpentyloxy, Methoxycarbonyl, Ethoxycarbonyl, Propoxycarbonyl, Isopropoxycarbonyl, Butoxycarbonyl, Isobutoxycycarbonyl, sec-Butoxycarbonyl, Pentyloxycarbonyl, Isopentyloxycarbonyl, Neopentyloxycarbonyl, tert-Pentyloxycarbonyl, Hexyloxycarbonyl, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, Mono- oder Dipentylcarbamoyl, Mono- oder Dihexylcarbamoyl oder N-Methyl-N-ethylcarbamoyl.

Reste X, L¹ und L² sind z.B. CH₂, (CH₂)₂, (CH₂)₃, (CH₂)₄, CH(CH₃)CH₂ oder CH(CH₃)CH(CH₃).

Reste X sind weiterhin z.B. (CH₂)₅, (CH₂)₆, (CH₂)₇, (CH₂)₈, C₂H₄―O―C₂H₄, C₂H₄―NH―C₂H₄, C₂H₄―N(CH₃)―C₂H₄, C₂H₄―O―C₂H₄―O―C₂H₄, C₂H₄―NH―C₂H₄―NH―C₂H₄, C₂H₄―N(CH₃)―C₂H₄―N(CH₃)―C₂H₄, C₂H₄―O―C₂H₄―N(CH₃)―C₂H₄, C₂H₄―O―C₂H₄―O―C₂H₄―O―C₂H₄, C₂H₄―NH―C₂H₄―NH―C₂H₄―NH―C₂H₄, C₂H₄―N(CH₃)―C₂H₄―N(CH₃)―C₂H₄―N(CH₃)―C₂H₄, C₂H₄―CO―NH-C₂H₄, C₂H₄―CO―N(CH₃)―C₂H₄, C₃H₆―CO―NH―C₂H₄, C₂H₄―CO―NH―C₃H₆, C₃H₆―CO―NH―C₃H₆ oder C₃H₆―CO―N(CH₃)―C₃H₆.

Der Rest Q steht für Hydroxy oder eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, Phenylsulfonyloxy, C₁-C₄-Alkanoyloxy, C₁-C₄-Dialkylamino oder ein Rest der Formel 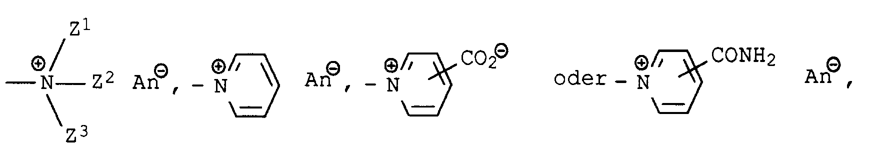
wobei Z¹, Z² und Z³ gleich oder verschieden sind und unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methylsulfonat, Phenylsulfonat oder 2- oder 4-Methylphenylsulfonat in Betracht kommen.

Bevorzugt sind Schwefelverbindungen der Formel I, in der A Methylen, Carbonyl oder den Rest der Formel CH₂―CO bedeutet.

Bevorzugt sind weiterhin Schwefelverbindungen der Formel I, in der X C₁-C₈-Alkylen, das gegebenenfalls durch 1 bis 3 Sauerstoffatome in Etherfunktion unterbrochen ist, bedeutet.

Bevorzugt sind weiterhin Schwefelverbindungen der Formel I, in der R¹ Wasserstoff oder Amino und R² Wasserstoff bedeuten.

Bevorzugt sind weiterhin Schwefelverbindungen der Formel I, in der n 2 bedeutet.

Besonders bevorzugt sind Schwefelverbindungen der Formel I, in der A Methylen bedeutet.

Besonders bevorzugt sind weiterhin Schwefelverbindungen der Formel I, in der R¹ Amino und R² Wasserstoff bedeuten.

Die neuen Schwefelverbindungen der Formel I können auf an sich bekannte Weise erhalten werden. Beispielsweise kann man ein Benzolderivat der Formel II 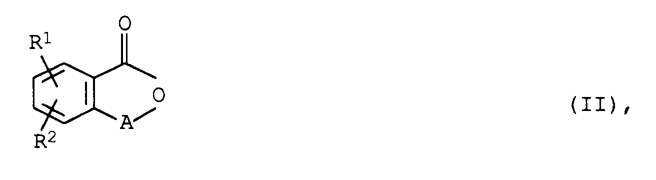
in der R¹, R² und A jeweils die obengenannte Bedeutung besitzen, mit einem Amin der Formel III

H₂N―X―S(O)ₙ―Y (III),

in der n, X und Y jeweils die obengenannte Bedeutung besitzen, umsetzen.

Vorteilhaft ist die Umsetzung der Benzolderivate der Formel II zunächst mit einem Thioether der Formel IIIa

H₂N―X―S―Y (IIIa),

in der X und Y jeweils die obengenannte Bedeutung besitzen, und anschließende Oxidation des Sulfids zur Sulfonylgruppe, z.B. mit Wasserstoffperoxid.

Die Reste R¹ und R², sofern sie von Wasserstoff verschieden sind, können auf an sich bekanntem Weg auch nachträglich in den Benzolring eingeführt werden. Insbesondere bei der Einführung der Hydroxysulfonylgruppe oder der Nitrogruppe, die anschließend zur Aminogruppe reduziert werden kann, ist dieser Weg bevorzugt.

Die neuen Schwefelverbindungen der Formel I sind wertvolle Zwischenprodukte für die Herstellung von Farbstoffen, insbesondere von Reaktivfarbstoffen.

Die folgenden Beispiele sollen die Erfindung näher erläutern.

### Beispiel 1

Eine Mischung aus 302 g 2-Aminoethyl-2'-hydroxyethylsulfid und 268 g Phthalid wurde 3 h auf 220 bis 225°C erhitzt. Nach dem Abkühlen gab man 250 ml Wasser zu und stellte den pH-Wert mit Schwefelsäure auf 6,5. Nach Zugabe von 1 g Wolframsäure tropfte man bei 65 bis 80°C 680 g 30 gew.-%iges wäßriges Wasserstoffperoxid innerhalb 1 h zu. Nach dem Abkühlen wurde der Niederschlag abgesaugt und getrocknet. Man isolierte 407 g der Verbindung der Formel 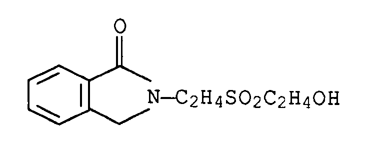
Fp.: 172 bis 174°C
NMR (D₆-DMSO) 3,31 (m,2), 3,52 (m,2), 3,81 (m,2), 4,00 (m,2), 4,55 (s,2), 5,20 (t,1), 7,5-7,7 (m,4).

### Beispiel 2

Man trug unter Kühlung 174 g der Verbindung aus Beispiel 1 in 600 g 96 gew.-%ige Schwefelsäure ein und rührte 8 h bei Raumtemperatur. Nach Abkühlen auf 0 bis 5°C gab man innerhalb 3 h 34,7 g 100 gew.-%ige Salpetersäure in 70 g 96 gew.-%iger Schwefelsäure zu. Nach weiteren 3 h wurde mit Eis verdünnt, der pH-Wert mit festem Natriumhydrogencarbonat auf 0,2 bis 0,3 gestellt und abgesaugt. Man isolierte 240 g der Verbindung der Formel 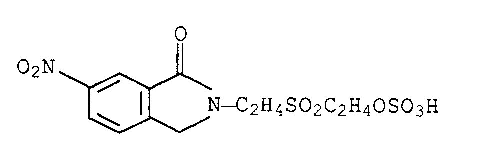
in Form eines feuchten Preßkuchens.

Fp.: 154 bis 157°C (aus Butanol).

### Beispiel 3

240 g des feuchten Preßkuchens aus Beispiel 2 wurden in 700 ml Wasser gelöst, der pH-Wert mit Natriumacetat auf 4,5 eingestellt und in Gegenwart von 5 g eines Palladiumkatalysators bei 50 bis 55°C mit Wasserstoff begast. Nach beendeter Wasserstoffaufnahme wurde vom Katalysator abfiltriert und das Filtrat unter vermindertem Druck eingeengt. Man isolierte nach Trocknung 177 g einer Verbindung der Formel 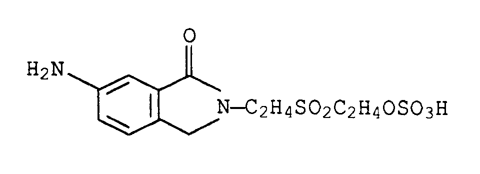
Gehaltsbestimmung durch Diazotierung: 69 %.
NMR (D₆-DMSO): 3,50 (m,4), 3,92 (m,2), 4,12 (m,2), 4,38 (s,2), 5,30 (s,2), 6,8 (m,2), 7,2 (d,1)

### Beispiel 4

Eine Lösung von 69 g der Verbindung aus Beispiel 3 in 500 ml Wasser wurde bei 25 bis 30°C mit Natriumcarbonat auf einen pH-Wert von 10 gestellt. Es schied sich ein Öl ab, das langsam kristallisierte. Man saugte ab und isolierte nach dem Trocknen 42 g der Verbindung 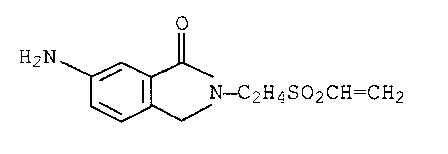
Fp.: 144 bis 148°C.
NMR (D₆-DMSO): 3,55 (m,2), 3,88 (m,2), 4,35 (s,2), 5,38 (s,2), 6,3 (m,2), 6,85 (m,2), 7.02 (m,1), 7,21 (d,1)

### Beispiel 5

135 g der Verbindung aus Beispiel 1 wurden in 500 g Xylol angerührt und bei 65 bis 68°C langsam mit 119 g Thionylchlorid versetzt. Nach weiteren 3 h bei 65 bis 68°C wurde abgekühlt und abgesaugt. Man isolierte nach Trocknen 144 g der Verbindung der Formel 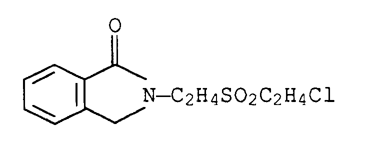
Fp.: 162 bis 164°C.

Man verfuhr dann weiter wie in Beispiel 2 und 3 beschrieben und erhielt die Verbindung der Formel 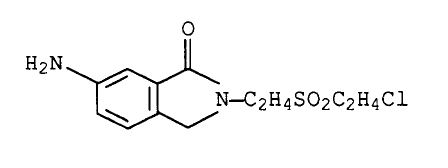
Fp.: 169 bis 171°C.

### Beispiel 6

Man verfuhr wie in Beispiel 1 beschrieben, ersetzte jedoch das Phthalid durch 296 g Phthalsäureanhydrid. Man erhielt 410 g der Verbindung der Formel 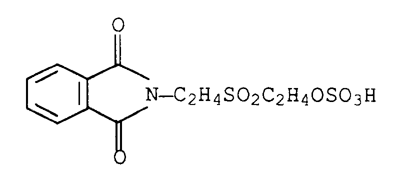
Fp.: 126 bis 130°C.

Man verfuhr dann weiter wie in Beispiel 2 und 3 beschrieben und erhielt die Verbindung der Formel 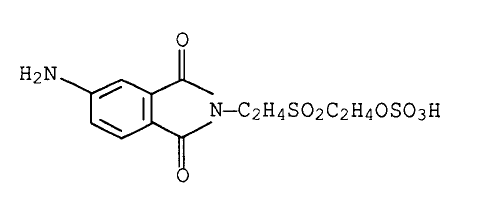
NMR (D₆-DMSO): 3,45 (m,4), 3,95 (t,2), 4,10 (t,2), 6,5 (s,2), 6,80 (dd,1), 6,95 (s,1), 7,50 (d,1)

### Beispiel 7

Die Verbindung aus Beispiel 6 wurde analog Beispiel 4 umgesetzt. Man erhielt so die Verbindung der Formel 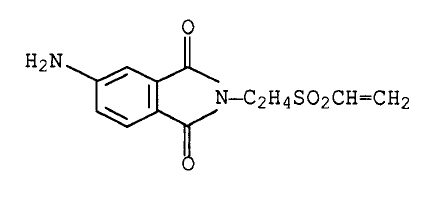
Fp.: 143 bis 145°C.

### Beispiel 8

Homophthalsäureanhydrid wurde wie in Beispiel 1 beschrieben mit 2-Aminoethyl-2'-hydroxyethylsulfid umgesetzt und das erhaltene Öl mit 30 gew.-%igem wäßrigem Wasserstoffperoxid oxidiert. Man isolierte so die Verbindung der Formel 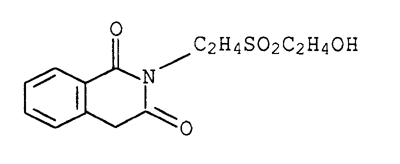
Fp.: 146 bis 149°C
NMR (D₆-DMSO): 3,4 (m,4), 3,80 (m,2), 4,15 (s,2), 4,30 (m,2), 5,21 (t,1), 7,41 (m,1), 7,50 (m,1), 7,68 (m,1), 8,08 (m,1)

## Patentansprüche

1. Schwefelverbindungen der Formel I 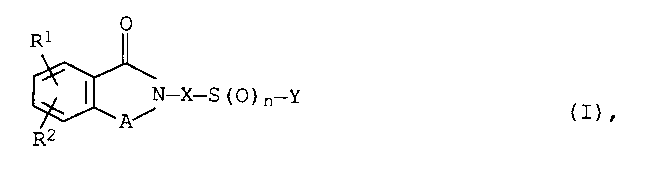 in der
n 0 oder 2,
R¹ und R² unabhängig voneinander jeweils Wasserstoff, C₁-C₆-Alkyl, C₁-C₆-Alkoxy, Halogen, Nitro, Amino, Hydroxysulfonyl, C₁-C₆-Alkoxycarbonyl, Carbamoyl oder C₁-C₆-Mono- oder Dialkylcarbamoyl,
A Methylen, Carbonyl, Sulfonyl oder einen Rest der Formel CH₂―CO oder CH₂-SO₂, wobei die Methylengruppe jeweils an den Benzolring geknüpft ist,
X eine direkte Bindung, C₁-C₈-Alkylen, das gegebenenfalls durch 1 bis 3 Sauerstoffatome in Etherfunktion, 1 bis 3 Iminogruppen oder 1 bis 3 C₁-C₄-Alkyliminogruppen unterbrochen ist, oder einen Rest der Formel L¹―CO―NR³―L², worin L¹ und L² unabhängig voneinander jeweils für C₁-C₄-Alkylen und R³ für Wasserstoff, C₁-C₄-Alkyl oder Phenyl stehen, und
Y Vinyl oder einen Rest der Formel C₂H₄Q, worin Q für Hydroxy oder eine unter alkalischen Reaktionsbedingungen abspaltbare Gruppe steht, bedeuten.

2. Schwefelverbindungen nach Anspruch 1, dadurch gekennzeichnet, daß A Methylen, Carbonyl oder den Rest der Formel CH₂―CO bedeutet.

3. Schwefelverbindungen nach Anspruch 1, dadurch gekennzeichnet, daß X C₁-C₈-Alkylen, das gegebenenfalls durch 1 bis 3 Sauerstoffatome in Etherfunktion unterbrochen ist, bedeutet.

4. Schwefelverbindungen nach Anspruch 1, dadurch gekennzeichnet, daß R¹ Wasserstoff oder Amino und R² Wasserstoff bedeuten.

5. Schwefelverbindungen nach Anspruch 1, dadurch gekennzeichnet, daß n 2 bedeutet.

6. Verfahren zur Herstellung von Schwefelverbindungen gemäß Anspruch 1, dadurch gekennzeichnet, daß man ein Benzolderivat der Formel II 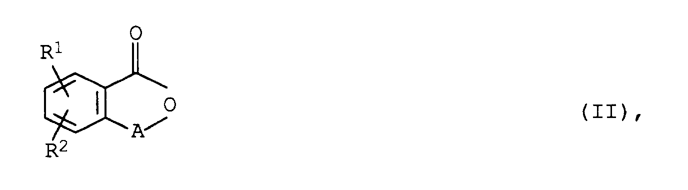 in der R¹, R² und A jeweils die in Anspruch 1 genannte Bedeutung besitzen, mit einem Amin der Formel III
H₂N―X―S(O)ₙ―Y (III),
in der n, X und Y jeweils die in Anspruch 1 genannte Bedeutung besitzen, umsetzt.
